# FASCICULE DE BREVET EUROPEEN

(11) **EP 3 809 915 B1**
(45) Date de publication et mention de la délivrance du brevet: **05.06.2024**
(21) Numéro de dépôt: 19745696.5
(22) Date de dépôt: 20.06.2019
(51) Int. Cl.: A45D 33/00, A45D 33/02

(54) **ENSEMBLE DE CONDITIONNEMENT DE PRODUIT COSMÉTIQUE ET UN PROCÉDÉ DE RÉALISATION D'UN TEL ENSEMBLE DE CONDITIONNEMENT DE PRODUIT COSMETIQUE**
VERPACKUNGSANORDNUNG FÜR KOSMETISCHE PRODUKTE UND VERFAHREN ZUR HERSTELLUNG EINER SOLCHEN VERPACKUNGSANORDNUNG FÜR KOSMETISCHE PRODUKTE
COSMETIC PRODUCT PACKAGING ASSEMBLY AND A METHOD FOR PRODUCING SUCH A COSMETIC PRODUCT PACKAGING ASSEMBLY

(30) Priorité: 21.06.2018 FR 1855526
(43) Date de publication de la demande: 28.04.2021
(73) Titulaire: L V M H RECHERCHE, 45800 St. Jean de Braye (FR)
(72) Inventeur: CHEVALIER, Marc, 95130 Franconville (FR)
(74) Mandataire: Plasseraud IP
(86) Numéro de dépôt international: PCT/FR2019/051512
(87) Numéro de publication internationale: WO 2019/243748

(56) Documents cités:
- EP-A2- 1 314 373
- WO-A1-01/76410
- WO-A1-97/28718
- WO-A1-2018/007758
- FR-A1- 2 860 960
- US-A- 3 570 036
- US-A- 4 698 871

## Description

L'invention concerne un ensemble de conditionnement de produit cosmétique comprenant un support absorbant. L'invention vise également un procédé de réalisation d'un tel ensemble de conditionnement de produit cosmétique.

### Art antérieur

Par « produit cosmétique », on entend au sens de la présente invention, un produit cosmétique destiné à être appliqué sur un être humain. Un « produit cosmétique » est plus généralement un produit tel que défini dans le règlement (CE) N° 1223/2009 du Parlement Européen et du Conseil, daté du 30 novembre 2009, relatif aux produits cosmétiques.

Le document FR 2 860 960 concerne un applicateur comportant une face d'application formée par un matériau comportant une pluralité d'alvéoles s'ouvrant sur la face d'application via au moins un bord débouchant, la face d'application étant au moins en partie recouverte d'un revêtement de flocage, formé de fibres, la taille moyenne des alvéoles étant telle que la surface sur laquelle sont implantées les fibres de flocage est de profil différent du profil d'une surface enveloppe de la face d'application contenant les bords débouchant des alvéoles.

La demande FR 3 039 378 décrit un exemple d'ensemble de conditionnement d'un produit cosmétique comprenant un support absorbant apte à être imprégné de produit cosmétique. Un tel support absorbant évite notamment tout écoulement intempestif du produit cosmétique, ce dernier étant relargué par mise en contact d'un applicateur avec le support absorbant et pression sur le support absorbant.

Selon FR 3 039 378, le support absorbant est une éponge à cellules ouvertes ou en un matériau à fibres tissées ou non-tissées. Ces matériaux présentent un bon pouvoir d'imprégnation de produit cosmétique. Cependant, ils ne permettent généralement de redistribuer ou relarguer qu'une partie du produit cosmétique dont ils sont imprégnés. Il s'en suit une perte de produit cosmétique importante, qui ne peut pas être utilisé.

Plus généralement, il existe un besoin pour un support absorbant pour un ensemble de conditionnement d'un produit cosmétique, dont les caractéristiques - notamment le pouvoir absorbant et le taux de restitution - peuvent être déterminées plus précisément.

### Résumé de l'invention

La présente invention concerne un ensemble de conditionnement de produit cosmétique selon la revendication 1 et un procédé de fabrication d'un tel ensemble de conditionnement de produit cosmétique selon la revendication 11. Les caractéristiques préférées de l'invention sont exposées dans les revendications dépendantes.

À cette fin, il est décrit le support absorbant déformable pour l'ensemble de conditionnement d'un produit cosmétique, le support absorbant présentant des mailles définies par des arêtes, les arêtes étant réalisées par fabrication additive.

Ainsi, avantageusement, la réalisation d'un tel support absorbant, notamment par un procédé de fabrication additive, permet d'obtenir des caractéristiques beaucoup plus fines et mieux contrôlées, que celles d'un support absorbant réalisé en matériau éponge ou en matériau textile tissé ou non tissé.

Selon des modes de réalisation préférés, le support absorbant comporte une ou plusieurs des caractéristiques suivantes, prises seules ou en combinaison :
- les arêtes sont réalisées par frittage sélectif au laser, par fusion sélective au laser, par stéréolitographie ou par dépôt fil tendu ;
- le support s'étendant selon une direction principale, les arrêtes sont conformées pour s'étendre sensiblement dans un même plan lorsque le support absorbant est comprimé par une pression dans la direction principale ;
- les arêtes sont reliées entre elles par des articulations, également réalisées par fabrication additive, de préférence concomitamment aux arêtes, le cas échéant ;
- le support s'étendant selon une direction principale, le support absorbant présente une première couche dans laquelle le support absorbant a des premières mailles et une deuxième couche, dans laquelle le support absorbant a des deuxièmes mailles, différentes des premières mailles ;
- les premières et deuxièmes mailles sont :
   - de formes différentes ; et/ou
   - de volumes différents ;
- le support absorbant s'étendant selon une direction principale, le support absorbant présente au moins une couche, orientée perpendiculairement à la direction principale, ladite au moins une couche ayant des troisièmes mailles et des quatrièmes mailles, les quatrièmes mailles étant différentes des troisièmes mailles ;
- les troisièmes et quatrièmes mailles sont :
   - de formes différentes ; et/ou
   - de volumes différents ;
- le support comprend sur au moins une de ses surfaces une peau, la peau présentant de préférence des parties étanches, la peau étant de préférence encore étanche sur toute sa surface, la peau étant monobloc avec le reste du support absorbant ou étant rapportée sur le reste du support absorbant ;
- le support absorbant étant de forme sensiblement cylindrique, s'étendant selon une direction principale d'extension, une peau latérale est réalisées sur la face latérale du support absorbant, la peau ayant de préférence une hauteur, mesurée selon la direction principale d'extension du support absorbant, sensiblement égale à la hauteur du support absorbant comprimé dans la direction principale d'extension ;
- le support absorbant comprend en outre une gouttière sur la surface latérale, conformée pour que la peau latérale soit reçue au moins partiellement dans la gouttière lorsque le support absorbant est comprimé dans la direction principale d'extension, la gouttière étant monobloc avec le reste du support absorbant ou étant rapporté sur le reste du support absorbant ;
- le support absorbant présente des mailles entremêlées, avec ou sans contact entre les mailles entremêlées ;
- les mailles ont une forme choisie parmi :
   - un polyèdre régulier, notamment un octaèdre régulier ; et
   - un polyèdre régulier tronqué, notamment un octaèdre régulier tronqué ;
- les arêtes des mailles ont une longueur comprise entre 0,05 mm et 5 mm ;
- les arêtes des mailles ont un diamètre compris entre 0,02 mm et 3 mm ;
- le support absorbant comprend au moins l'un parmi :
   - des mailles ouvertes ;
   - des mailles semi-ouvertes ; et
- le support absorbant comprend en outre des mailles fermées.

L'ensemble de conditionnement d'un produit cosmétique comprend au moins un réceptacle destiné à recevoir le produit cosmétique, un support absorbant tel que décrit ci-avant dans toutes ses combinaisons, reçu dans le réceptacle, et un couvercle pour fermer le réceptacle.

L'ensemble de conditionnement comprend en outre un boîtier, recevant le réceptacle, un élément applicateur du produit cosmétique et un capot pour fermer le boîtier.

Il est encore décrit une recharge de produit cosmétique pour un ensemble de conditionnement d'un produit cosmétique, en particulier tel que décrit ci-avant dans toutes ses combinaisons, comprenant un support absorbant tel que décrit ci-avant dans toutes ses combinaisons, imprégné de produit cosmétique dans un sachet fermé de manière étanche.

Le support absorbant peut être reçu dans un réceptacle, de préférence fermé par un couvercle, à l'intérieur du sachet.

Le procédé de fabrication d'un ensemble de conditionnement d'un produit cosmétique tel que décrit ci-avant, dans toutes ses combinaisons, comprenant les étapes consistant à :
- réaliser un support absorbant en mettant en oeuvre un procédé comprenant au moins une étape a) de réalisation des arêtes des mailles du support absorbant par fabrication additive, les arêtes des mailles étant de préférence réalisées à l'étape a) par impression 3D, notamment par SLS, FSL, stéréolithographie ou par dépôt de fil,
- fournir au moins un réceptacle destiné à recevoir le produit cosmétique, et un couvercle apte à fermer le réceptacle,
- disposer le support absorbant dans le réceptacle,
- imprégner le support absorbant de produit cosmétique, et
- fermer le réceptacle avec le couvercle.

Le procédé de fabrication peut comprendre en outre les étapes consistant à :
- fournir un boîtier et un capot apte à fermer le boîtier,
- fournir un élément applicateur de produit cosmétique,
- disposer le réceptacle et l'élément applicateur de produit cosmétique dans le boîtier, et
- fermer le boîtier à l'aide du capot.

### Brève description des figures

L'invention sera mieux comprise de la description qui suit, faite au regard des dessins ci-annexés, parmi lesquels :
- la figure 1 représente schématiquement en coupe un ensemble de conditionnement d'un produit cosmétique avec un support absorbant ;
- la figure 2 illustre l'utilisation de l'ensemble de conditionnement d'un produit cosmétique de la figure 1 ;
- les figures 3 à 5 illustrent des exemples de mailles que peut présenter le support absorbant de la figure 1 ; et
- la figure 6 illustre schématiquement un réseau de mailles que peut présenter le support absorbant de la figure 1 ;
- la figure 7 illustre schématiquement des sections possibles des arrêtes des mailles du support absorbant de la figure 1 ;
- la figure 8 représente schématiquement deux mailles possible du support absorbant de la figure 1 ;
- la figure 9 illustre schématiquement un deuxième exemple de réseau de mailles que peut présenter le support absorbant de la figure 1 ;
- les figures 10 à 14 illustrent des variantes du support absorbant de la figure 1 ;
- la figure 15 illustre schématiquement en coupe un exemple de recharge de produit cosmétique pour l'ensemble de conditionnement de produit cosmétique de la figure 1 ; et
- la figure 16 est un ordinogramme d'un procédé de fabrication de l'ensemble de conditionnement de la figure 1.

### Description détaillée

Dans la suite de la description, les éléments identiques ou de fonction identique portent le même signe de référence. À fin de concision de la présente description, ces éléments ne sont pas décrits dans le cadre de chaque mode de réalisation. Au contraire, seules les différences entre les modes de réalisation sont décrites.

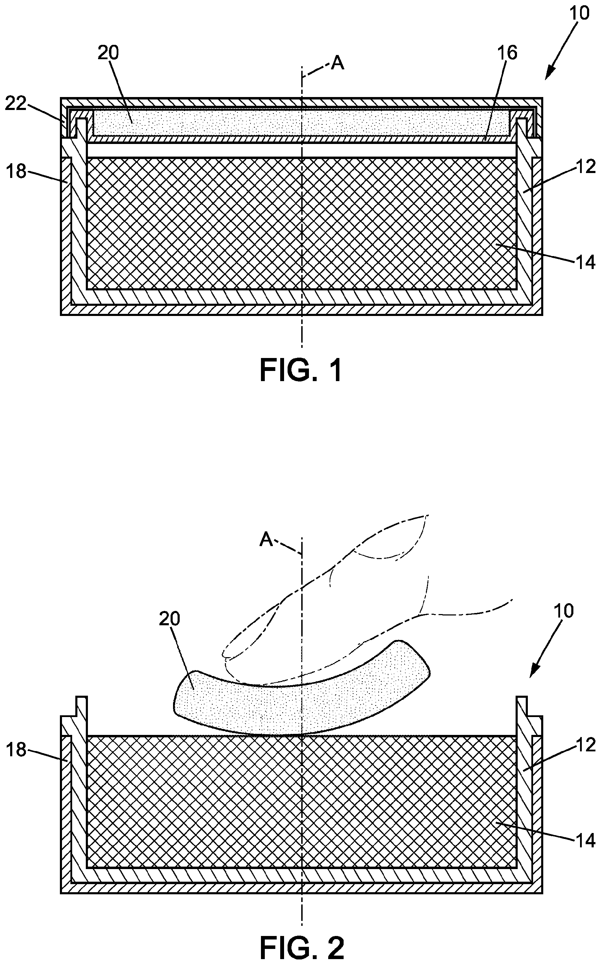

La figure 1 illustre un exemple 10 d'ensemble de conditionnement d'un produit cosmétique. Tel qu'illustré, l'ensemble de conditionnement 10 comprend un réceptacle 12, un support absorbant 14, reçu dans le réceptacle 12, et un couvercle 16 pour fermer le réceptacle 12.

Par « support absorbant », on entend ici un support adapté à retenir du produit cosmétique, par exemple par capillarité. Le produit cosmétique peut notamment être fluide, en particulier liquide, plus particulièrement encore visqueux, ou sous forme pulvérulente.

Le support absorbant 14 est adapté pour être imprégné de produit cosmétique. De préférence, le support absorbant 14 est imprégné de produit cosmétique - c'est-à-dire qu'il retient du produit cosmétique.

Le support absorbant 14 est déformable (ou flexible). Notamment le support absorbant est avantageusement comprimable au moyen d'une pression manuelle, par exemple à l'aide d'un pouce, d'un utilisateur, avantageusement pour restituer au moins une partie du produit cosmétique dont il est imprégné. Par comprimable, on entend ici qu'on peut minimiser le volume du support absorbant, notamment par pression manuelle par un utilisateur, en particulier en appliquant une pression avec le pouce, le cas échéant à travers un élément applicateur à charger en produit cosmétique à partir du produit cosmétique retenu dans le support absorbant 14.

La compression du support absorbant permet de restituer au moins une partie du produit cosmétique dont il est imprégné. Notamment, à partir d'un support absorbant initialement imprégné à saturation de produit cosmétique, il est de préférence possible de restituer plus de 80%, de préférence encore plus de 90% du produit cosmétique dont le support absorbant est initialement imprégné, en minimisant le volume du support absorbant par compression.

En outre, ici, l'ensemble de conditionnement 10 comprend également un boîtier 18 recevant le réceptacle 12, un élément applicateur de produit cosmétique 20 et un capot 22 pour fermer le boîtier 18. Ici, l'élément applicateur 20 est logé dans le couvercle 16 fermant le réceptacle 12. Le réceptacle 12 est par exemple reçu dans le boîtier 18 de manière amovible. Il est alors possible de remplacer le réceptacle 12 dans le boîtier 18 et, ainsi, de recharger l'ensemble de conditionnement 10 en produit cosmétique. Selon un exemple, dans ce cas, l'élément applicateur 20 peut également être remplacé, de préférence, concomitamment avec le réceptacle 12.

L'élément applicateur 20 est par exemple une éponge.

Le réceptacle 12 est disposé selon un axe principal A, vertical en utilisation, qui coïncide avec l'axe d'extension principal du support absorbant 14.

Dans l'exemple illustré, le support absorbant 14 est de forme sensiblement cylindrique, d'axe d'extension A. Ici, le support absorbant a une forme cylindrique de révolution. La section transversale du support absorbant 14 peut prendre d'autres formes que celle d'un disque. De manière générale, la forme du support absorbant 14 peut être quelconque.

Le couvercle 20 peut être fixé sur le réceptacle 12 par tout moyen approprié accessible à l'homme de l'art. Notamment, le couvercle 20 peut être vissé ou clipsé sur le réceptacle 12. Également, le couvercle 20 peut être articulé sur le réceptacle 12, au moyen d'une charnière (non illustrée) et/ou muni de moyens de verrouillage (non illustrés) du couvercle 20 dans sa position fermant le réceptacle 12.

De même, le capot 22 peut être fixé au boitier 18, directement ou indirectement par tout moyen accessible à l'homme de l'art. Le capot 22 peut ainsi, en particulier, être clipsé ou vissé soit directement sur le boîtier 18, soit sur le couvercle 16 du réceptacle 12. Le capot 22 peut également être monté sur le boitier 18 ou sur le couvercle 16 du réceptacle 12 au moyen d'une charnière et/ou être muni de moyens de verrouillage (non illustrés) du capot 22 dans sa position fermant le boîtier 18.

Bien entendu, d'autres configurations de l'ensemble de conditionnement 10 sont également possibles.

Comme cela est visible sur la figure 2, l'utilisation d'un tel ensemble de conditionnement 10 se fait en appliquant l'élément applicateur 20 contre le support absorbant 14, de manière que le produit cosmétique dont le support absorbant 14 est imprégné, soit relargué et imprègne l'élément applicateur 20. L'utilisateur peut alors appliquer le produit cosmétique dont l'élément applicateur 20 est imprégné, par exemple sur sa peau.

Le produit cosmétique peut notamment être un fond de teint liquide, un fond de teint semi-liquide, un fond de teint sous forme de poudre ou tout autre substance cosmétique ou de soin, telle que du parfum, du maquillage. Le produit cosmétique peut en particulier être choisi parmi ceux nécessitant d'être distribués à travers un tamis, d'une grille, d'une peau. Le produit cosmétique peut également être choisi parmi ceux qui nécessitent d'être réhomogénéisés comme un gloss, un auto-bronzant, un parfum intégrant une poudre, des nacres ou des particules. Le produit cosmétique peut encore être choisi parmi les formules de soin comme par exemple :
- les formules dites « scrub », qui comprennent des éléments abrasifs leur donnant un effet exfoliant, ou
- les formules de soin multiphasiques, notamment du type « eau dans huile », « huile dans eau » ou « eau dans silicone », par exemple, ou
- les formules démaquillantes multi-phases.

Le produit cosmétique peut encore être choisi parmi les formules intégrant des tensioactifs, qui peuvent également servir à la génération de mousse grâce au support absorbant.

Les exemples indiqués ci-dessus sont non exhaustifs et ne forment pas une liste limitative de produit et d'utilisation.

L'intérêt du support absorbant 14 est ainsi, en premier lieu, de retenir le produit cosmétique plus ou moins visqueux voire pulvérulent, dans le réceptacle 12, et ainsi d'éviter les fuites ou plus généralement les pertes de produit cosmétique.

Cependant, un autre intérêt du support absorbant 14 peut être de mélanger le produit cosmétique, juste avant le relargage du produit cosmétique et son application. Par exemple, le produit cosmétique peut être composé d'éléments insolubles et présenter, au repos, la forme d'une émulsion avec différentes phases. Le produit cosmétique peut en particulier comporter des particules, solides, dans un fluide, et/ou plusieurs fluides de densités différentes. Dans ce cas, une ou plusieurs pressions sur le support absorbant 14, dans la direction de l'axe d'extension principale A du support absorbant 14, permet(tent) de mélanger les différentes phases et/ou de répartir les particules dans le produit cosmétique. Le produit cosmétique appliqué a ainsi une texture plus homogène.

Ici, de manière remarquable, le support absorbant 14 présente des mailles 24, définies par des arêtes 26 réalisées par fabrication additive. Une maille 24 est définie par une pluralité d'arêtes 26. Par maille, on entend ici un motif géométrique formé par des arêtes et qui se répète de manière à former un réseau. Le réseau peut être régulier, si toutes les mailles sont identiques, ou irrégulier si les mailles varient.

De manière connue en soi, on entend par « fabrication additive », les procédés de fabrication par ajout de matière. La fabrication additive est avantageusement assistée par ordinateur. Une fabrication additive peut notamment être réalisée par mise en forme d'une pièce par ajout de matière, par exemple par empilement de couches successives. Ainsi, la fabrication additive s'oppose notamment aux procédés de retrait de matière ou de moulage imposant des contraintes géométriques à leur mise en oeuvre, ou aux procédés résultant de réactions chimiques, comme ceux généralement mis en oeuvre pour réaliser une matière éponge, et dont le résultat est une structure aléatoire et/ou irrégulière.

La fabrication additive est parfois appelée « impression tridimensionnelle » ou « impression 3D », notamment par le grand public.

Un tel procédé de fabrication additive permet de réaliser de nombreuses variantes du support absorbant 14, présentant un grand nombre de caractéristiques avantageuses par rapport à un support en matériau éponge ou en textile tissé ou non-tissé.

De nombreux procédés peuvent être mis en oeuvre pour réaliser les arêtes 26 des mailles 24 du support absorbant 14, notamment :
- frittage sélectif par laser (ou SLS, pour « Sélective Laser Sintering » en anglais) : selon ce procédé, on réalise un modèle en trois dimensions par couches successives en deux dimensions, d'épaisseur choisie, un laser balayant chaque couche successivement dans un bac de fine poudre et la frittant ;
- fusion sélective par laser (ou SLM pour « Selctive Laser Melting », en anglais) : selon ce procédé, on réalise un modèle en trois dimensions par couches successives en deux dimensions, d'épaisseur choisie, un laser balayant chaque couche successivement dans un bac de fine poudre et la faisant fondre. La solidification du modèle a lieu directement après l'arrêt du laser ;
- stéréolitographie (ou SLA pour « Stereolithography apparatus » en anglais) : selon ce procédé, on réaliser un modèle en trois dimensions par couches successives en deux dimensions, d'épaisseur choisie, un couple de lasers balayant chaque couche successivement dans un bain de liquide et le polymérisant uniquement à l'intersection des deux faisceaux lasers ;
- dépôt de fil (ou FDM pour « Fused déposition modeling » en anglais) : selon ce procédé, on réalise un modèle en trois dimensions par dépôt d'un fil fondu.

Ces procédés de fabrication sont notamment choisis en fonction du ou des matériaux mis en oeuvre et/ou des dimensions souhaitées pour les arêtes 26 et/ou les mailles 24.

Dans le cas du support absorbant 14, celui-ci peut notamment être en l'un parmi :
- un polyamide, par exemple PA11,
- un polyéthylène,
- un polyuréthane thermoplastique ou TPU,
- un polyarylethertones ou PAEK, et
- un métal ou un alliage de métaux qui peu(ven)t notamment être choisi(s) parmi :
   - l'aluminium ou un alliage d'aluminium,
   - le cobalt ou un alliage de cobalt,
   - le chrome ou un alliage de chrome, notamment un alliage de cobalt et de chrome,
   - le nickel ou un alliage de nickel,
   - un acier inoxydable, et
   - le titane ou un alliage de titane.

Avec un procédé de fabrication additive, il est ainsi possible de réaliser des mailles 24 qui peuvent retenir le produit cosmétique. Pour ce faire, le volume de chaque maille 24 est réduit. Le volume de chaque maille est adapté à la formule à contenir. La fabrication additive permet notamment d'adapter le format de la maille.

Ainsi le volume des mailles 24 est adapté à la formule qu'elles contiennent, notamment afin de leur permettre de pouvoir stocker le produit cosmétique, par exemple par capillarité.

Les arêtes 26 des mailles 24 ont par exemple une longueur 126 compris entre 0,5 mm à 5 mm. La longueur des arêtes 26 des mailles 24 est choisie, dans cet intervalle, en fonction de la formule contenue.

Également, les arêtes des mailles ont par exemple un diamètre d26 compris entre 0,02 mm et 3 mm. Par « diamètre d'une arête », on entend ici la plus grande dimension de la section transversale d'une arête.

La forme des mailles 24 peut être très variée. Ces mailles 24 peuvent en pratique prendre toute forme réalisable par un procédé de fabrication additive permettant au support absorbant 14 de stocker le produit cosmétique, puis de le relarguer par pression sur le support absorbant 14.

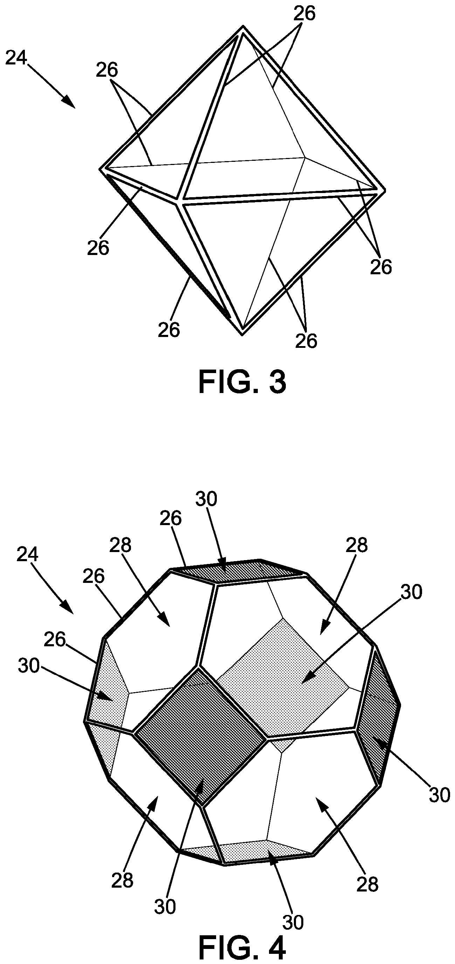

Notamment, les mailles 24 peuvent être polyédriques. Les figures 3 et 4 illustrent des exemples de telles mailles.

Sur la figure 3, la maille 24 a la forme d'un octaèdre. Ici, par exemple, la maille est ouverte, c'est-à-dire que l'espace entre les arêtes 26 est vide.

Sur la figure 3, la maille 24 a la forme d'un octaèdre tronqué. Ici, la maille 24 représentée est semi-ouverte, c'est-à-dire que certaines surfaces 28 de l'octaèdre tronqué formé par les arêtes 26 sont vides alors que d'autres surfaces 30 de l'octaèdre tronqué formé par les arêtes 26 sont pleines.

De manière générale et indépendamment de la forme des mailles 24, celles-ci peuvent être soit ouvertes, soit fermées, soit partiellement ouvertes (ou semi-ouvertes). Également, dans un même support absorbant 14, on peut réaliser des mailles ouvertes et/ou des mailles fermées et/ou des mailles semi-ouvertes. Il est à noter ici qu'avec un procédé de fabrication additive, il est possible de définir très précisément les mailles ouvertes, fermées ou semi-ouvertes. La réalisation de mailles fermées et/ou semi-fermées peut notamment permettre de guider le flux de produit cosmétique. Par exemple, les mailles fermées et/ou semi-fermées sont conformées pour diriger le flux de produit cosmétique, en cas de pression sur le support absorbant selon l'axe A, dans la direction de l'axe A, vers les extrémités longitudinales. Alternativement ou au surplus, les mailles fermées et/ou semi-fermées sont conformées pour dévier le flux de produit cosmétique, en cas de pression sur le support absorbant selon l'axe A, d'une direction menant vers la surface latérale du support absorbant 14.

Selon un mode de réalisation particulièrement avantageux, les mailles 24 présentent une forme telle que les arêtes qui la définissent peuvent être sensiblement dans un même plan quand le support absorbant 14 est comprimé selon la direction d'extension A. Ceci présente l'avantage de limiter le « volume mort » des mailles 24 dans lequel du produit cosmétique peut rester emprisonné quand on comprime le support absorbant 14.

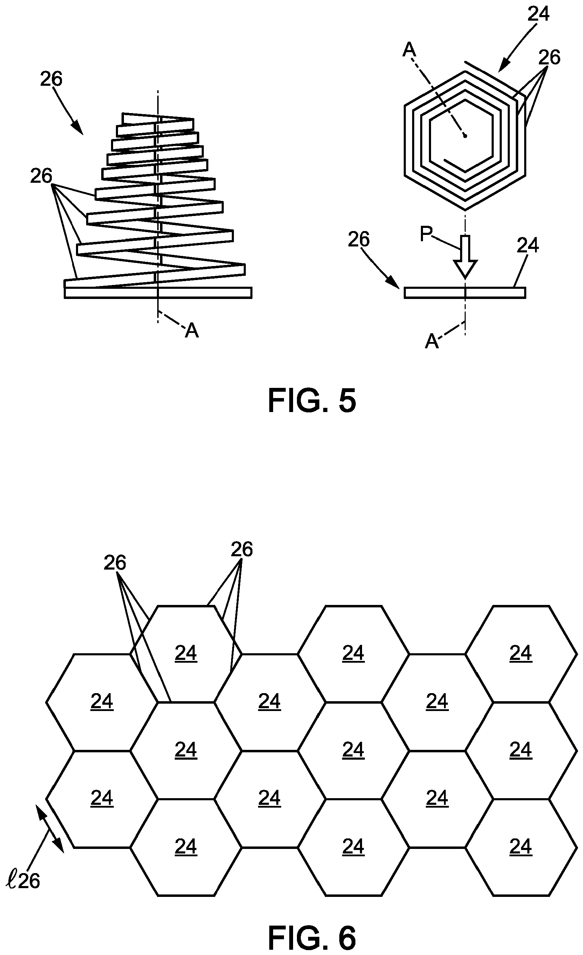

Un exemple d'une telle maille 24 est illustré à la figure 5. Sur cette figure, une maille 24 est délimitée par des arêtes 26 s'étendant selon une forme d'hélice tronconique d'axe A. Ainsi, en cas de compression de la maille 24, par pression P selon la direction de l'axe d'extension A de la maille 24, celle-ci se retrouve réduite à une forme de spirale, dans un plan.

Comme illustrée à la figure 6, les mailles 24 peuvent toutes être identiques. Les mailles 24 peuvent également être accolées les unes aux autres, par exemple en partageant des arêtes 26 avec des mailles 24 voisines. Alternativement, cependant, les mailles 24 peuvent être distantes les unes des autres.

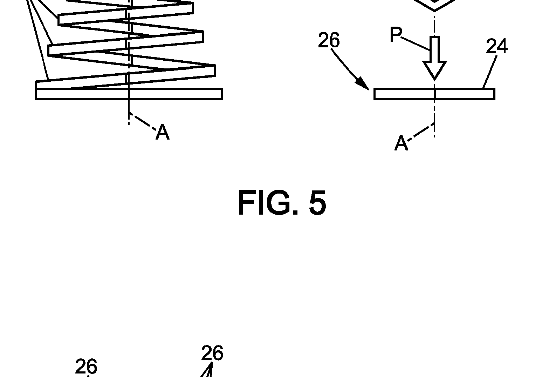

Comme cela est visible à la figure 7, les arêtes 26 sont cylindriques, de section 26s polygonale, notamment triangulaire, carrée ou hexagonale, ou ronde ou oblongue. La section des arêtes 26 peut notamment être choisie pour améliorer le mélange réalisé au moyen du support absorbant 14, en cas de compression de celui-ci. Pour ce faire, une section transversale polygonale apparait préférée. Au contraire, une section transversale ne présentant pas d'angle apparait préférable pour augmenter le volume de produit cosmétique que le support absorbant peut relarguer quand on le comprime.

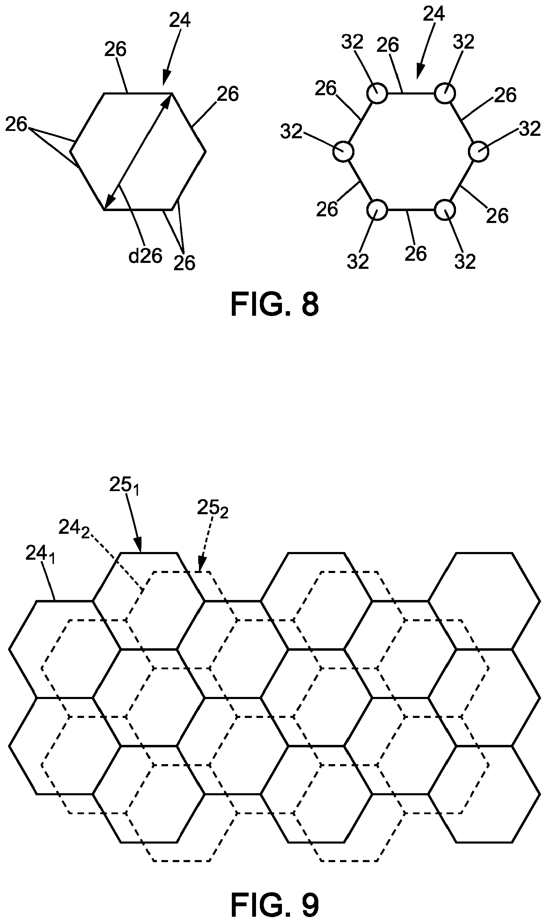

Également, les arêtes 26 peuvent être de section sensiblement constante sur toute leur longueur et être reliées entre elles directement, comme illustré à gauche sur la figure 8. Par directement, on entend ici sans interposition d'un élément de structure. Dans ce cas, la flexibilité du support absorbant 14 est donnée par la flexibilité des arêtes 26 elles-mêmes. En d'autres termes, pour assurer que le support absorbant 14 puisse relarguer du produit cosmétique, il convient de dimensionner les arrêtes 26 de manière que celles-ci puissent se déformer quand un utilisateur applique une pression P sur le support absorbant 14, par exemple avec le pouce, directement ou à travers l'élément applicateur 20.

Au contraire, comme illustré sur la droite de la figure 8, les arêtes 26 peuvent être reliées entre elles par des articulations 32. Les articulations 32 peuvent notamment être réalisées par des portions de section réduite, notamment de section inférieure à la section des arêtes 26. Les articulations sont également réalisées par un procédé de fabrication additive, par exemple concomitamment aux arêtes 26 des mailles 24 du support absorbant 14. Dans ce cas, il est possible d'obtenir la flexibilité du support absorbant du seul fait des articulations 32, les arêtes 26 étant assez rigides pour ne pas se déformer en cas de pression P d'un utilisateur.

Alternativement, la flexibilité du support absorbant 14 est obtenue par la combinaison d'une flexibilité des arêtes 26 et la présence d'articulations 32 entre ces arêtes 26.

Par ailleurs, comme cela est visible schématiquement sur la figure 9, les mailles 24 peuvent former deux réseaux 25₁, 25₂ de mailles 24₁, 24₂ entremêlées. Les mailles 24₁, 24₂ des deux réseaux 25₁, 25₂ peuvent être indépendantes les unes des autres ou, au contraire, être reliées les unes aux autres. Des mailles entremêlées peuvent permettre d'optimiser les capacités de mélange de la formule, un tel mélange n'étant pas possible avec d'autres technologies. Également, des mailles entremêlées peuvent permettre d'optimiser la souplesse du support absorbant 14, en minimisant la résistance à la compression.

Ici, les deux réseaux 25₁, 25₂ sont sensiblement identiques, comprenant des mailles 24₁, 24₂ identiques. Alternativement, cependant, les deux réseaux 25₁, 25₂ peuvent avoir des formes différentes. Notamment, les mailles 24₁ du premier réseau 25₁ peuvent être différentes des mailles 24₂ du deuxième réseau 25₂.

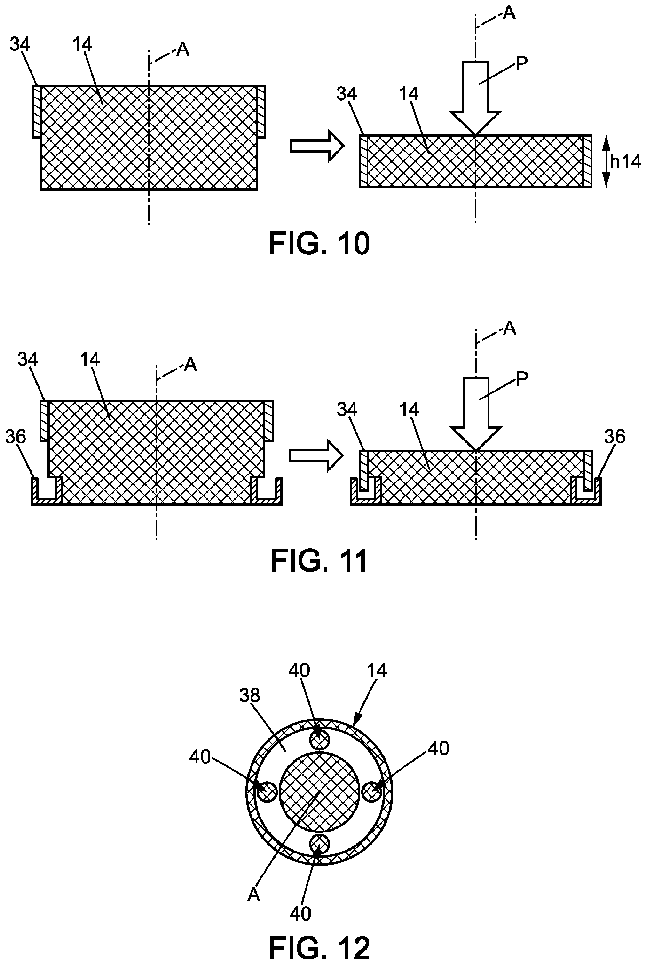

La figure 10 représente une vue en coupe d'un autre exemple de support absorbant 14. Celui-ci se distingue de celui qui vient d'être décrit essentiellement en ce qu'il comporte une peau 34. Cette peau 34 s'étend ici sur la surface latérale du support absorbant 14. Cette peau peut être réalisée séparément puis rapportée sur le support absorbant 14. Alternativement, cependant, cette peau 34 est réalisée concomitamment avec le reste du support absorbant 14, notamment avec les arêtes 26 des mailles 24.

Alternativement, la peau peut être en matériau de type textile, naturel comme synthétique, en tissu, naturel comme synthétique, en feutre, ou être de la peau de synthèse.

La peau 34 peut former un élément de filtration ou de régulation de la distribution de produit cosmétique, par exemple en empêchant ou, au contraire, en autorisant, la distribution de produit cosmétique uniquement localement.

Ici cette peau 34 est étanche (ou imperméable). La peau 34 s'étend, comme cela est illustré à la figure 10, sur une hauteur h14 correspondant à la hauteur minimale du support absorbant 14 lorsqu'il est comprimé sous l'effet d'une pression P orientée selon l'axe A. ainsi, cette peau permet de limiter la quantité de produit cosmétique qui s'échappe par la face latérale du support absorbant 14 lorsqu'un utilisateur le comprime dans la direction A. avantageusement, cette peau 34 est fixée sur le support absorbant 14 au voisinage de son extrémité supérieure, en utilisation.

La peau 34 peut être liée au support absorbant 14, en un ou plusieurs points ou sur la totalité de la hauteur de la peau 34. Notamment afin d'améliorer la propreté d'utilisation du produit cosmétique, le support absorbant 14 peut également être pourvu d'une couronne à structure fermée ou partiellement fermée, sur les premiers millimètres de la peau, formant ainsi une couronne autour du support absorbant 14. Ceci peut permettre de former un anneau de protection antisalissure sur la surface latérale du support absorbant, autour de la surface de distribution de la formule.

Sur la figure 11, le support absorbant présente, outre une peau 34, identique à celle présentée précédemment, une gouttière 36. La gouttière 36 présente essentiellement une forme annulaire à section en U. La gouttière 36 est ici fixée à l'extrémité inférieure du support absorbant 14, en utilisation. Là encore, la gouttière 36 peut être réalisée séparément du reste du support absorbant 14 puis rapportée sur celui-ci, ou concomitamment avec le reste du support absorbant, c'est-à-dire les arêtes 26 des mailles 24 et, de préférence, la peau 34, le cas échéant. Comme cela est visible sur la figure 11, la gouttière 36 est conformée pour recevoir au moins partiellement la peau 34 en cas de pression P sur le support absorbant 14 dans la direction A. Ici encore, la coopération de la peau 34 reçue dans la gouttière 36 vise à limiter voire à empêcher l'écoulement de produit cosmétique par la face latérale du support absorbant, en cas de pression P par un utilisateur, sur le support absorbant 14, dans la direction A. La coopération de la peau 34 et de la gouttière 36 augmente en effet la perte de charge à vaincre par le produit cosmétique pour pouvoir s'écouler à travers la face latérale du support absorbant 14.

Sur la figure 12, une peau 38 est disposée sur une surface extrémale - en l'espèce la surface supérieure - du support absorbant 14. Cette peau 38 est également étanche au produit cosmétique. Cette peau 38 peut également être réalisée séparément et être rapportée sur le support absorbant 14 ou être réalisée de manière concomitante avec le reste du support absorbant, notamment les arêtes 26 des mailles 24 et, de préférence, la peau 34 sur la surface latérale du support absorbant 14 et/ou la gouttière 36, le cas échéant. À titre d'exemple, la peau 38 illustrée à la figure 12 présente une forme annulaire et a des trous 40 traversant. Ainsi, la peau 38 limite le passage de produit cosmétique depuis les mailles 24 à la surface supérieure du support absorbant 14. Une telle peau 38 permet ainsi de contrôler la quantité de produit cosmétique distribuée par le support absorbant 14 quand il est comprimé selon sa direction A d'extension principale.

Alternativement ou au surplus, une peau 42 peut être réalisée, comme décrit précédemment, notamment à la surface supérieure du support absorbant 14. Cette peau 42 permet notamment d'obtenir un effet esthétique et/ou d'indiquer une marque, un texte, un logo, un dessin ou tout autre signe de communication. Comme précédemment indiqué, cette peau peut également jouer un rôle de « réducteur de débit » à la distribution de la formule contenue et ce dans un but d'optimisation de la restitution de la formule sur l'élément applicateur.

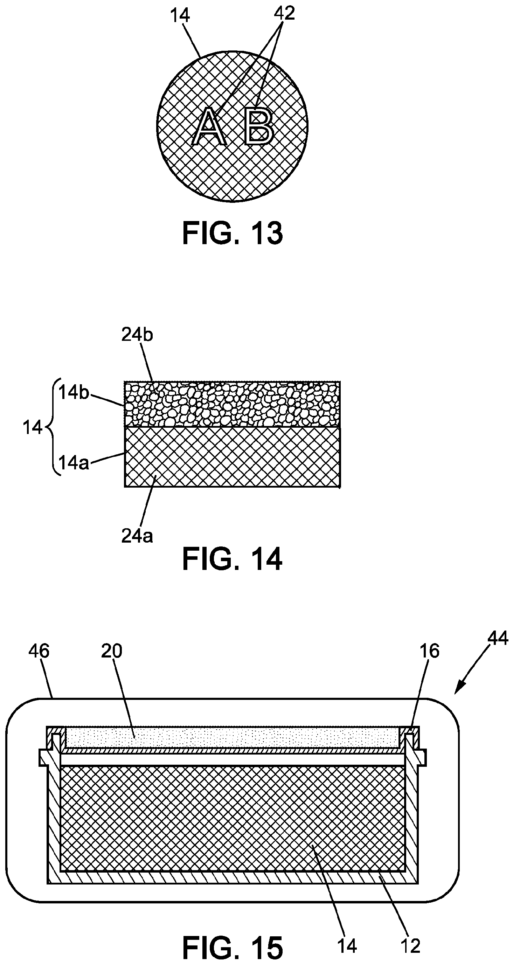

Comme illustré à la figure 14, le support absorbant 14 peut présenter une première couche 14a dans laquelle le support absorbant 14 a des premières mailles 24a et une deuxième couche 14b, dans laquelle le support absorbant 14 a des deuxièmes mailles 24b, différentes des premières mailles 24a. Notamment, les premières et deuxièmes mailles 24a, 24b sont de formes différentes et/ou de volumes différents. En particulier, les deuxièmes mailles 24b situées vers le haut du support absorbant 14 là où le produit cosmétique est prélevé, ont un volume plus petit que les premières mailles 24a.

Plus généralement, les mailles 24 du support absorbant 14 peuvent ne pas toutes être identiques. Les mailles 24 du support absorbant 14 peuvent notamment avoir un volume qui décroit, plus elles sont proches de la surface du support absorbant 14 par laquelle le produit cosmétique est prélevé.

Comme illustré à la figure 15, l'ensemble de conditionnement de produit cosmétique 10 peut être rechargé au moyen d'une recharge 44. Cette recharge de produit cosmétique 44 comporte, telle qu'illustrée à la figure 15, un support absorbant 14, tel que décrit précédemment, dans toutes ses variantes, imprégné de produit cosmétique dans un sachet 46 fermé de manière étanche. Ici, la recharge 44 comporte en outre un réceptacle 12, recevant le support absorbant 14, à l'intérieur du sachet 46, fermé par un couvercle 16 et recevant un élément applicateur 20.

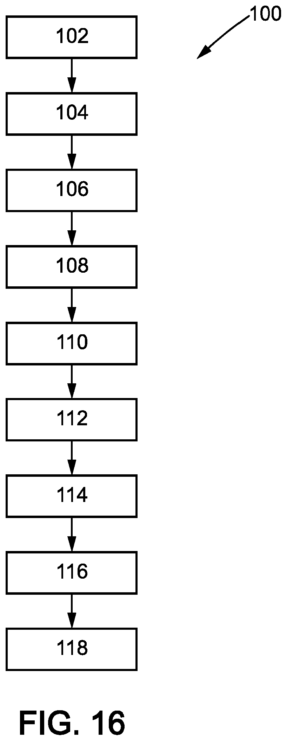

Par ailleurs, la figure 16 représente schématiquement un ordinogramme d'un procédé 100 de fabrication d'un ensemble de conditionnement de produit cosmétique 10.

Ce procédé de fabrication comprend une étape 102 de réalisation des arêtes 26 des mailles 24 du support absorbant 14 par fabrication additive.

Comme indiqué précédemment, le procédé de fabrication additive mis en oeuvre peut notamment être un procédé de frittage sélectif au laser, un procédé de fusion sélective au laser, un procédé de stéréolitographie ou un procédé par dépôt fil tendu.

Par ailleurs, le support absorbant 14 peut également être recouvert partiellement ou en totalité par un élément de filtration ou de régulation de la distribution de produit cosmétique, l'élément de régulation permettant ou, au contraire, empêchant la distribution de produit cosmétique, localement. Cet élément de filtration ou de régulation de la distribution de produit cosmétique, tout comme la peau 34 décrit précédemment, peut notamment être en matériau textile, naturel comme synthétique, en tissus, naturel comme synthétique, en feutre, ou en peau de synthèse. L'élément de filtration peut être fixé au support absorbant 14 ou non.

Le support absorbant 14 peut également être recouvert afin améliorer le rendu au touché pour un utilisateur.

Le procédé 100 se poursuit par une étape 104 de fourniture d'au moins un réceptacle 12 destiné à recevoir le produit cosmétique, et un couvercle 16 apte à fermer le réceptacle 12.

Il est à noter ici que le support absorbant 14 peut être recouvert avant, pendant ou après son intégration dans le réceptacle 12.

À l'étape 106, on dispose le support absorbant 14 dans le réceptacle 12, et, à l'étape 108, on imprègne le support absorbant 14 de produit cosmétique. À l'étape 110, on ferme le réceptacle 12 avec le couvercle 16.

On fournit alors, à l'étape 112, un boîtier 18 et un capot 22 apte à fermer le boitier 18. À l'étape 114, on fournit un élément applicateur 20 de produit cosmétique. À une étape 116, on dispose le réceptacle 12 et l'élément applicateur 20 de produit cosmétique dans le boîtier 18. Enfin, à l'étape 118, on ferme le boîtier 18 à l'aide du capot 20.

La géométrie des mailles est de préférence choisie de manière à minimiser le volume de celles-ci en cas de compression du support absorbant, limitant ainsi le volume mort du support absorbant.

Selon une variante non représentée, le support absorbant présente des mailles différentes, dans une même couche s'étendant perpendiculairement à l'axe A. La couche s'étendant perpendiculairement à l'axe A peut notamment correspondre à l'intégralité du volume du support d'absorption. Dans ce cas, les mailles dépendent uniquement de leur position radiale par rapport à l'axe A et sont indépendantes de leur position verticale, mesurée le long de l'axe A. Par exemple, les mailles sont de plus en plus petites, ou au contraire de plus en plus grande, dans la direction radiale, depuis l'axe A vers la surface latérale du support absorbant 14. De mailles de plus en plus petites en se rapprochant de la surface latérale du support absorbant 14 permet notamment d'augmenter progressivement la perte de charge du flux de produit cosmétique se rapprochant de la surface latérale du support absorbant. On peut ainsi limiter la quantité de produit cosmétique s'échappant du support absorbant 14 par la surface latérale.

Dans l'exemple décrit, le support absorbant 14 est mis en oeuvre en tant que réservoir/mélangeur de produit cosmétique.

## Revendications

1. Ensemble de conditionnement d'un produit cosmétique (10), comprenant au moins un réceptacle (12) destiné à recevoir le produit cosmétique, un support absorbant (14) reçu dans le réceptacle (12), et un couvercle (16) pour fermer le réceptacle (12), l'ensemble de conditionnement (10) comprenant en outre un boîtier (18), recevant le réceptacle (12), un élément applicateur du produit cosmétique (20) et un capot (22) pour fermer le boîtier (18), dans lequel le support absorbant (14) présentant des mailles (24) définies par des arêtes (26), les arêtes (26) étant cylindriques de section polygonale, notamment carrée ou hexagonale, ronde ou oblongue, les arêtes étant réalisées par fabrication additive, de préférence par frittage sélectif au laser, par fusion sélective au laser, par stéréolithographie ou par dépôt fil tendu.

2. Ensemble de conditionnement selon la revendication 1, dans lequel le support absorbant s'étend selon une direction principale (A), les arrêtes (26) étant conformées pour s'étendre sensiblement dans un même plan lorsque le support absorbant (14) est comprimé par une pression (P) dans la direction principale (A).

3. Ensemble de conditionnement selon la revendication 1 ou 2, dans lequel les arêtes (26) sont reliées entre elles par des articulations (32), également réalisées par fabrication additive, de préférence concomitamment aux arêtes (26), le cas échéant.

4. Ensemble de conditionnement, le support absorbant s'étendant selon une direction principale (A), dans lequel le support absorbant (14) présente une première couche (14a) dans laquelle le support absorbant (14) a des premières mailles (24a) et une deuxième couche (14b), dans laquelle le support absorbant (14) a des deuxièmes mailles (24b), différentes des premières mailles (24a), les premières et deuxièmes mailles (24a ; 24b) étant de préférence de formes différentes et/ou de volumes différents.

5. Ensemble de conditionnement selon l'une quelconque des revendications précédentes, le support absorbant s'étendant selon une direction principale (A), dans lequel le support absorbant (14) présente au moins une couche, orientée perpendiculairement à la direction principale (A), ladite au moins une couche ayant des troisièmes mailles et des quatrièmes mailles, les quatrièmes mailles étant différentes des troisièmes mailles, les troisièmes et quatrièmes mailles étant de préférence de formes différentes et/ou de volumes différents.

6. Ensemble de conditionnement selon l'une quelconque des revendications précédentes, le support absorbant comprenant sur au moins une de ses surfaces une peau (34 ; 38 ; 42), la peau (34 ; 38 ; 42) présentant de préférence des parties étanches, la peau (34 ; 38 ; 42) étant de préférence encore étanche sur toute sa surface, la peau (34 ; 38 ; 42) étant monobloc avec le reste du support absorbant ou étant rapportée sur le reste du support absorbant, le support absorbant étant de préférence de forme sensiblement cylindrique, s'étendant selon une direction principale d'extension (A), une peau latérale (34) étant de préférence réalisée sur la face latérale du support absorbant (14), la peau ayant de préférence une hauteur, mesurée selon la direction principale d'extension (A) du support absorbant (14), sensiblement égale à la hauteur (h14) du support absorbant (14) comprimé dans la direction principale d'extension (A), le support absorbant comprenant de préférence une gouttière (36) sur la surface latérale, conformée pour que la peau latérale (34) soit reçue au moins partiellement dans la gouttière (36) lorsque le support absorbant (14) est comprimé dans la direction principale d'extension (A), la gouttière (36) étant monobloc avec le reste du support absorbant (14) ou étant rapporté sur le reste du support absorbant (14).

7. Ensemble de conditionnement selon l'une quelconque des revendications précédentes, le support absorbant présentant des mailles (24₁, 24₂) entremêlées, avec ou sans contact entre les mailles (24₁, 24₂) entremêlées.

8. Ensemble de conditionnement selon l'une quelconque des revendications précédentes, dans lequel les mailles (24) ont une forme choisie parmi :
- un polyèdre régulier, notamment un octaèdre régulier ; et
- un polyèdre régulier tronqué, notamment un octaèdre régulier tronqué.

9. Ensemble de conditionnement selon l'une quelconque des revendications précédentes, dans lequel les arêtes (26) des mailles (24) ont une longueur (126) comprise entre 0,05 mm et 5 mm et/ou dans lequel les arêtes (26) des mailles (24) ont un diamètre (d26) compris entre 0,02 mm et 3 mm.

10. Ensemble de conditionnement selon l'une quelconque des revendications précédentes, le support absorbant comprenant au moins l'un parmi :
- des mailles (24) ouvertes ;
- des mailles semi-ouvertes ;
le support absorbant comprenant en outre de préférence des mailles fermées.

11. Procédé de fabrication d'un ensemble de conditionnement d'un produit cosmétique selon l'une quelconque des revendications précédentes, comprenant les étapes consistant à :
- réaliser un support absorbant (14) en mettant en oeuvre un procédé comprenant au moins une étape a) de réalisation des arêtes (26) des mailles (24) du support absorbant (14) par fabrication additive, les arêtes (26) des mailles (24) étant de préférence réalisées à l'étape a) par impression 3D, notamment par SLS, FSL, stéréolithographie ou par dépôt de fil,
- fournir au moins un réceptacle (12) destiné à recevoir le produit cosmétique, et un couvercle (16) apte à fermer le réceptacle (12),
- disposer le support absorbant (14) dans le réceptacle (12),
- imprégner le support absorbant (14) de produit cosmétique, et
- fermer le réceptacle (12) avec le couvercle (16),
le procédé comprenant en outre de préférence les étapes consistant à :
- fournir un boîtier (18) et un capot (22) apte à fermer le boîtier (18),
- fournir un élément applicateur de produit cosmétique (20),
- disposer le réceptacle (12) et l'élément applicateur de produit cosmétique (20) dans le boîtier (18), et
- fermer le boîtier (18) à l'aide du capot (22).

## Patentansprüche

1. Verpackungsanordnung (10) für ein Kosmetikprodukt, aufweisend mindestens einen zur Aufnahme des Kosmetikprodukts vorgesehenen Behälter (12), einen in dem Behälter (12) aufgenommenen absorbierenden Träger (14) und einen Deckel (16) zum Schließen des Behälters (12), wobei die Verpackungsanordnung (10) ferner ein den Behälter (12) aufnehmendes Gehäuse (18), ein Kosmetikprodukt-Applikatorelement (20) und eine Kappe (22) zum Schlie-βen des Gehäuses (18) aufweist,
in welcher der absorbierende Träger (14) von Kanten (26) definierte Maschen (24) aufweist, wobei die Kanten (26) zylindrisch sind, mit einem polygonalen, insbesondere quadratischen oder hexagonalen, runden oder ovalen Querschnitt, wobei die Kanten durch additive Fertigung hergestellt sind, vorzugsweise durch selektives Sintern mittels Laser, durch selektives Verschmelzen mittels Laser, durch Stereolithographie oder Ablegen eines gespannten Fadens.

2. Verpackungsanordnung nach Anspruch 1, in welcher der absorbierende Träger sich in einer Hauptrichtung (A) erstreckt, wobei die Kanten (26) gestaltet sind, um sich im Wesentlichen in einer gleichen Ebene zu erstrecken, wenn der absorbierende Träger (14) durch einen Druck (P) in der Hauptrichtung (A) zusammengepresst ist.

3. Verpackungsanordnung nach Anspruch 1 oder 2, in welcher die Kanten (26) untereinander durch Gelenke (32) verbunden sind, die ebenfalls durch additive Fertigung gebildet sind, vorzugsweise gegebenenfalls gleichzeitig mit den Kanten (26).

4. Verpackungsanordnung, wobei der absorbierende Träger sich in einer Hauptrichtung (A) erstreckt, in welcher der absorbierende Träger (14) aufweist: eine erste Schicht (14a), in welcher der absorbierende Träger (14) erste Maschen (24a) hat, und eine zweite Schicht (14b), in welcher der absorbierende Träger (14) zweite Maschen (24b) hat, die sich von den ersten Maschen (24a) unterscheiden, wobei die ersten und zweiten Maschen (24a; 24b) sich vorzugsweise bezüglich Form und/oder Volumen voneinander unterscheiden.

5. Verpackungsanordnung nach einem der vorstehenden Ansprüche, wobei der absorbierende Träger sich in einer Hauptrichtung erstreckt, in welcher der absorbierende Träger (14) mindestens eine Schicht aufweist, die senkrecht zur Hauptrichtung (A) orientiert ist, wobei die mindestens eine Schicht dritte Maschen und vierte Maschen aufweist, die vierten Maschen sich von den dritten Maschen unterscheiden, die dritten und vierten Maschen sich vorzugsweise bezüglich Form und/oder Volumen voneinander unterscheiden.

6. Verpackungsanordnung nach einem der vorstehenden Ansprüche, wobei der absorbierende Träger an mindestens einer seiner Oberflächen eine Haut (34; 38; 42) aufweist, die Haut (34; 38; 42) bevorzugt dichte Bereiche aufweist, die Haut (34; 38; 42) stärker bevorzugt dicht über ihre gesamte Oberfläche ist, die Haut (34; 38; 42) integral mit dem Rest des absorbierenden Trägers ist oder an den Rest des absorbierenden Trägers angefügt ist, der absorbierende Träger vorzugsweise im Wesentlichen zylinderförmig ist und sich in einer Haupterstreckungsrichtung (A) erstreckt, vorzugsweise eine seitliche Haut (34) an der seitlichen Oberfläche des absorbierenden Trägers (14) gebildet ist, die Haut vorzugsweise eine in der Haupterstreckungsrichtung (A) des absorbierenden Trägers (14) gemessene Höhe hat, die im Wesentlichen gleich der Höhe (h14) des in der Haupterstreckungsrichtung (A) zusammengepressten absorbierenden Trägers (14) ist, der absorbierende Träger an der seitlichen Oberfläche vorzugsweise eine Rinne aufweist, die derart gestaltet ist, dass die seitliche Haut (34) mindestens teilweise in der Rinne (36) aufgenommen ist, wenn der absorbierende Träger (14) in der Haupterstreckungsrichtung (A) zusammengepresst ist, wobei die Rinne (36) integral mit dem Rest des absorbierenden Trägers (14) ist oder an den Rest des absorbierenden Trägers (14) angefügt ist.

7. Verpackungsanordnung nach einem der vorstehenden Ansprüche, wobei der absorbierende Träger miteinander vermischte Maschen (24₁, 24₂) aufweist, mit oder ohne Kontakt zwischen den vermischten Maschen (24₁, 24₂).

8. Verpackungsanordnung nach einem der vorstehenden Ansprüche, in welcher die Maschen (24) eine Form haben, die ausgewählt ist aus:
- einem regelmäßigen Polyeder, insbesondere einem regelmäßigen Oktaeder; und
- einem regelmäßigen Polyederstumpf, insbesondere einem regelmäßigen Oktaederstumpf.

9. Verpackungsanordnung nach einem der vorstehenden Ansprüche, in welcher die Kanten (26) der Maschen (24) eine Länge (126) zwischen 0,05mm und 5mm haben und/oder in welcher die Kanten (26) der Maschen (24) einen Durchmesser (d26) zwischen 0,02mm und 3mm haben.

10. Verpackungsanordnung nach einem der vorstehenden Ansprüche, wobei der absorbierende Träger aufweist:
- offene Maschen (24); und/oder
- halboffene Maschen;
wobei der absorbierende Träger ferner vorzugsweise geschlossene Maschen aufweist.

11. Verfahren zur Herstellung einer Verpackungsanordnung für ein Kosmetikprodukt nach einem der vorstehenden Ansprüche, aufweisend die folgenden Schritte:
- Bilden eines absorbierenden Trägers (14) durch Ausführen eines Verfahrens, das mindestens einen Schritt a) zum Bilden von Kanten (26) von Maschen (24) des absorbierenden Trägers (14) durch additive Fertigung aufweist, wobei die Kanten (26) der Maschen (24) vorzugsweise im Schritt a) durch 3D-Druck, insbesondere durch SLS, FSL, Stereolithographie oder durch Fadenablegen gebildet werden,
- Bereitstellen mindestens eines zur Aufnahme des Kosmetikprodukts vorgesehenen Behälters (12) und eines geeigneten Deckels (16) zum Schließen des Behälters (12),
- Anordnen des absorbierenden Trägers (14) in dem Behälter (12),
- Tränken des absorbierenden Trägers (14) mit Kosmetikprodukt, und
- Schließen des Behälters (12) mit dem Deckel (16),
wobei das Verfahren vorzugsweise ferner die folgenden Schritte aufweist:
- Bereitstellen eines Gehäuses (18) und einer zum Schließen des Gehäuses (18) geeigneten Kappe (22),
- Bereitstellen eines Kosmetikprodukt-Applikatorelements (20),
- Anordnen des Behälters (12) und des Kosmetikprodukt-Applikatorelements (20) in dem Gehäuse (18), und
- Schließen des Gehäuses (18) mithilfe der Kappe (22).

## Claims

1. Packaging assembly for a cosmetic product (10), comprising at least one receptacle (12) intended to receive the cosmetic product, an absorbent substrate (14) received in the receptacle (12), and a cover (16) for closing the receptacle (12), the packaging assembly (10) further comprising a housing (18), receiving the receptacle (12), a cosmetic product applicator member (20), and a cap (22) for closing the housing (18), wherein the deformable absorbent substrate (14) having cells (24) defined by ridges (26), the ridges being created by additive manufacturing, preferably by selective laser sintering, selective laser melting, stereolithography, or fused filament fabrication.

2. Packaging assembly according to claim 1, wherein the absorbent substrate extends in a main direction (A), the ridges (26) being shaped to extend substantially in a same plane when the absorbent substrate (14) is compressed by pressure (P) in the main direction (A).

3. Packaging assembly according to claim 1 or 2, wherein the ridges (26) are interconnected by hinges (32), also created by additive manufacturing, preferably concurrently with the ridges (26), where appropriate.

4. Packaging assembly, the absorbent substrate extending in a main direction (A), wherein the absorbent substrate (14) has a first layer (14a) in which the absorbent substrate (14) has first cells (24a) and a second layer (14b) in which the absorbent substrate (14) has second cells (24b) different from the first cells (24a), the first and second cells (24a; 24b) are preferably of different shapes and/or of different volumes.

5. Packaging assembly according to any of the preceding claims, the absorbent substrate extending in a main direction (A), wherein the absorbent substrate (14) has at least one layer, oriented perpendicularly to the main direction (A), said at least one layer having third cells and fourth cells, the fourth cells being different from the third cells, the third and fourth cells are preferably of different shapes and/or of different volumes.

6. Packaging assembly according to any of the preceding claims, the absorbent substrate comprising a skin (34; 38; 42) on at least one of its surfaces, the skin (34; 38; 42) preferably having fluidtight portions, the skin (34; 38; 42) more preferably being fluidtight over its entire surface, the skin (34; 38; 42) being integral with the rest of the absorbent substrate or being attached to the rest of the absorbent substrate, the absorbent substrate preferably of substantially cylindrical shape, extending in a main direction of extension (A), wherein a side skin (34) is preferably created on the side face of the absorbent substrate (14), the skin preferably having a height, measured along the main direction of extension (A) of the absorbent substrate (14), that is substantially equal to the height (h14) of the absorbent substrate (14) compressed in the main direction of extension (A), the absorbent substrate preferably comprising a channel (36) on the side surface, shaped so that the side skin (34) is at least partially received in the channel (36) when the absorbent substrate (14) is compressed in the main direction of extension (A), the channel (36) being integral with the rest of the absorbent substrate (14) or being attached to the rest of the absorbent substrate (14).

7. Packaging assembly according to any of the preceding claims, the absorbent substrate having interlinked cells (24₁, 24₂), with or without contact between the interlinked cells (24₁, 24₂).

8. Packaging assembly according to any of the preceding claims, wherein the cells (24) have a shape selected from:
- a regular polyhedron, in particular a regular octahedron; and
- a regular truncated polyhedron, in particular a regular truncated octahedron.

9. Packaging assembly according to any of the preceding claims, wherein the ridges (26) of the cells (24) have a length (*l*26) between 0.05 mm and 5 mm and/or the ridges (26) of the cells (24) have a diameter (d26) between 0.02 mm and 3 mm.

10. Packaging assembly according to any of the preceding claims, the absorbent substrate comprising at least one among:
- open cells (24);
- semi-open cells,
the absorbent substrate preferably further comprising cells that are closed.

11. Method for manufacturing a packaging assembly for a cosmetic product according to any of the preceding claims, comprising the steps consisting of:
- producing an absorbent substrate (14) by implementing a method comprising at least a step a) of creating the ridges (26) of the cells (24) of the absorbent substrate (14) by additive manufacturing, the ridges (26) of the cells (24) are preferably created in step a) by 3D printing, in particular by SLS, FSL, stereolithography, or fused filament fabrication,
- providing at least one receptacle (12) intended to receive the cosmetic product, and a cover (16) suitable for closing the receptacle (12),
- placing the absorbent substrate (14) in the receptacle (12),
- impregnating the absorbent substrate (14) with cosmetic product, and
- closing the receptacle (12) with the cover (16)
the method further comprising preferably the steps consisting in:
- providing a housing (18) and a cap (22) suitable for closing the housing (18),
- providing a cosmetic product applicator member (20),
- arranging the receptacle (12) and the cosmetic product applicator member (20) in the housing (18), and
- closing the housing (18) using the cap (22).
